# EUROPEAN PATENT SPECIFICATION

(11) **EP 1 442 129 B1**
(45) Date of publication and mention of the grant of the patent: **10.09.2008**
(21) Application number: 02804820.5
(22) Date of filing: 18.10.2002
(51) Int. Cl.: G01N 33/68, C12Q 1/68

(54) **CANCER MONITORING BY ABERRANT PROMOTOR METHYLATION OF THE TRANSCRIPTION FACTOR GENES PAX5 ALPHA PAX5 BETA, NOVEL LOOP HELIX LOOP PROTEIN, NOVEL GENE 2, AND BETA3 GENES**
ÜBERWACHUNG VON KREBS DURCH PROMOTOR-FEHLMETHYLIERUNG DER TRANSKRIPTIONSFAKTORGENE PAX5 ALPHA PAX5 BETA, NEUES SCHLEIFE-HELIX-SCHLEIFE-PROTEIN, NEUES GEN 2, SOWIE BETA3-GENE
SURVEILLANCE DU CANCER PAR METHYLATION DE PROMOTEUR ABERRANT DES GENES DE FACTEUR DE TRANSCRIPTION PAX5 ALPHA ET PAX5 BETA, NOUVELLE PROTEINE BOUCLE-HELICE-BOUCLE, NOUVEAU GENE 2, ET GENES BETA 3

(30) Priority: 18.10.2001 US 348407 P
(43) Date of publication of application: 04.08.2004
(73) Proprietor: Lovelace Respiratory Research Institute, Albuquerque, NM 87108-5127 (US)
(72) Inventor: PALMISANO, William, A., Edgewood, NM 87015 (US); BELINSKY, Steven, A., Albuquerque, NM 87112 (US)
(74) Representative: Kupecz, Arpad
(86) International application number: PCT/US2002/033499
(87) International publication number: WO 2003/064682

(56) References cited:
- WO-A-02/068694
- WO-A1-99/63110
- US-A- 6 017 704
- CHIUBACHI T ET AL: "Hypermethylation of the human Pax-5 gene promoter" BLOOD, vol. 94, no. 10 SUPPL. 1 PART 2, 15 November 1999 (1999-11-15), page 296b, XP008053446 & FORTY-FIRST ANNUAL MEETING OF THE AMERICAN SOCIETY OF HEMATOLOGY; NEW ORLEANS, LOUISIANA, USA; DECEMBER 3-7, 1999 ISSN: 0006-4971
- BUSSLINGER M ET AL: "Deregulation of PAX-5 by translocation of the Emu enhancer of the IgH locus adjacent to two alternative PAX-5 promoters in a diffuse large-cell lymphoma." PROCEEDINGS OF THE NATIONAL ACADEMY OF SCIENCES OF THE UNITED STATES OF AMERICA. 11 JUN 1996, vol. 93, no. 12, 11 June 1996 (1996-06-11), pages 6129-6134, XP002347784 ISSN: 0027-8424
- DANBARA M ET AL: "DNA methylation dominates transcriptional silencing of Pax5 in terminally differentiated B cell lines" MOLECULAR IMMUNOLOGY, ELMSFORD, NY, US, vol. 38, 2001, pages 1161-1166, XP002965066 ISSN: 0161-5890
- DANBARA M. ET AL.: 'DNA methylation dominates transcriptional silencing of Pax5 in terminally differentiated B cell lines' MOLECULAR IMMUNOLOGY vol. 38, 2001, pages 1161 - 1166, XP002965066

## Description

### CROSS-REFERENCE TO RELATED APPLICATIONS

This application claims the benefit of the filing of United States of America provisional patent application Serial No. 60/348,407, entitled "Inactivation of PAX5 Alpha and Beta Genes in Cancer," filed on October 18, 2001.

### GOVERNMENT RIGHTS

The U.S. Government has certain rights this invention and the right in limited circumstances to require the patent owner to license others on reasonable terms as provided for by the terms of Contract No. DAMD17-99-1-9258 awarded by U.S. Department of Defense.

### BACKGROUND

### Field of the Invention (Technical Field):

The present invention relates to methods of monitoring for cancer according to claims 1 and 2.

### Background Art:

The prognosis for patients with cancer is primarily dependent on the stage of the tumor at the time of clinical diagnosis. Human neoplasms are known to arise through a progressive accumulation of genetic alterations in protooncogenes and tumor suppressor genes. Because these genetic changes are retained as a tumor grows, they can serve as markers for detection of cancer. Therefore, it is useful that these genetic markers be identified, so they can be used for early detection, to monitor therapy, and to predict outcome of the disease.

Drs. James Herman and Stephen Baylin developed a technique known as methylation specific polymerase chain reaction (MSP) to detect gene-specific alterations in promoter methylation within tumor cells. This technique, described in U.S. Patent Nos. 5,786,146 and 6,017,704, has been used to detect promoter hypermethylation of cancer genes (e.g., p16, Rb ER, and MGMT) and can detect one copy of the methylated gene in a background of 1,000 unmethylated copies.

There is now evidence linking dysregulation of the DNA methylation machinery with tumorigenesis and tumor progression. In neoplasia, DNA methylation patterns increase within the promoter of many tumor suppressor genes. This alteration leads to gene silencing, and serves as an alternative to coding region mutations. Recently, a technique called methylated CpG island amplification was developed as a genome screening approach to identify genes that are differentially methylated in cancer cells. See Patent Cooperation Treaty Application No. PCT/US01/26452, filed August 24, 2001, assigned to the assignee of the present invention.

A need remains for an identification of the novel genes inactivated by promoter hypermethylation in cancers, such as human breast cancer, and to determine the commonality for gene inactivation in other solid tumor types. Against this background, the present invention was developed.

Chiubachi T. et al., Blood 94 (10 Suppl.), 1999, p296B, XP008053446, discloses hypermethylation of the human Pax-5 gene promoter in human multiple myeloma.

### SUMMARY OF THE INVENTION (DISCLOSURE OF THE INVENTION)

PAX5 α and β genes, and others identified, as markers for detecting, monitoring, and diagnosing human cancer. A polymerase chain reaction-based technique of methylated CpG island amplification, followed by representational difference analysis, identifies these genes methylated in human cancer.

Aberrant methylation of 5'CpG islands is a key epigenetic event in many human cancers. Two of CpG islands are identified and mapped to the 5' UTR region of the PAX5 α and β genes. These genes, located on chromosome 9p13, are transcribed from two distinct promoters and form two alternative first exons that are subsequently spliced to the common exons 2-10. The resulting splice variants encode two distinct transcription factors important in cell differentiation and embryonic development. Examination of the methylation status of each gene using methylation-specific PCR reveals that both genes are hypermethylated in, for example, breast, lung and colon tumors. Analysis of methylated cell lines and tumors by combined bisulfite restriction analysis and bisulfite sequencing reveals dense methylation patterns within each 5'CpG island, strongly correlating with transcriptional silencing. The identified aberrant promoter methylation is a mechanism for dysregulation of the PAX5 and other identified genes.

Objects, advantages and novel features, and further scope of applicability will be set forth in part in the detailed description to follow, taken in conjunction with the accompanying drawings, and in part will become apparent to those skilled in the art upon examination of the following, or may be learned by practice of the methods. The objects and advantages of the methods may be realized and attained by means of the instrumentalities and combinations particularly pointed out in the appended claims.

### BRIEF DESCRIPTION OF THE DRAWINGS

The accompanying drawings, which are incorporated into and form a part of the specification, illustrate several embodiments of the present invention and, together with the description, serve to explain the principles of the invention. The drawings are only for the purpose of illustrating a preferred embodiment of the invention and are not to be construed as limiting the invention. In the drawings:
Fig. 1 is a table showing the frequency of PAX5 α and PAX5 β methylation in tumor cell line primary tumors and non-malignant specimens;
Fig. 2 illustrates a representative methylation-specific polymerase chain reaction analysis according to the present invention;
Figs. 3A and 3B illustrate a confirmation, by methylation-specific polymerase chain reaction analysis, of methylation changes indicative of PAX5 gene inactivation, whereby PAX5 α transcript was abundant or present in certain cell lines (Fig. 3A), while no transcript was present in other cell lines (Fig. 3B);
Figs. 4A and 4B are bar graphs summarizing methylation density, as a percent of CpG sites methylated, of PAX5 α and PAX5 β genes, respectively, in tumor cell line primary tumors and non-malignant specimens, according to the invention, indicative of the methylation and silencing of the PAX5 α and PAX5 β genes; and
Fig. 5 is a table showing the frequency of methylation of the Novel Helix Loop Helix Binding Protein, Novel Gene 2, and Beta3 genes in tumor cell lines, primary tumors and non-malignan specimens.

### DESCRIPTION

Breast cancer is the second leading cause of cancer-related death in the United States, and the number of cases is increasing each year. Mortality from this disease could be reduced greatly through an improved understanding of the molecular alterations contributing to cancer initiation and progression. A mechanism in many sporadic cancers, aberrant promoter hypermethylation, is an epigenetic event involving the methylation of normally unmethylated cytosines within the promoter region of genes **(Baylin and Herman, 2000; Jones and Laird, 1999).** This change in methylation pattern leads to transcriptional silencing and serves as an alternative to coding region mutation (**Baylin and Herman, 2000; Jones and Laird, 1999).** In breast cancer, aberrant methylation inactivates numerous genes functioning in key cellular pathways (**Yang *et al.,* 2001; Zochbauer-Muller *et al.,* 2001; Du *et al.,* 2001; Burbee; *et al.,* 2001; Toyooka *et al.,* 2001).**

Cancer genome-wide screening approaches are known, for identifying genes inactivated by promoter hypermethylation. These approaches include methylation-sensitive arbitrarily primed PCR **(Gonzalgo *et al.,*1997; Huang *et al.,*1997),** restriction landmark genomic scanning **(Costello *et al.,* 2000),** CpG microarrays **(Yan *et al.,* 2001),** methyl-CpG binding domain chromatography **(Brock *et al.,* 2001),** and methylated CpG island amplification (MCA) coupled with representational difference analysis (RDA) **(Toyota *et al.,*1999a).** The MCA/RDA approach has been used to identify several methylated genes involved in colorectal **(Toyota *et al.,* 1999b; Toyota *et al.,* 1999c)** and pancreatic cancers **(Ueki *et al.,* 2001).** MCA/RDA is a PCR/subtraction hybridization based assay that allows for the rapid amplification and selection of densely methylated CpG rich regions ranging in size from 200 bp to 2 kb.

The PAX5 gene plays a role in cell differentiation and embryonic development, and is located on chromosome 9p13. This locus is frequently associated with chromosomal translocations and contains two distinct promoters resulting in two alternative 5' exons (α and β) that are spliced to common coding sequences of exons 2-10. Using amplicons from the breast cancer cell line MCF7 as the tester, and amplicons from normal breast tissue as the driver, the applicants identified the region 5' UTR and exon 1 of the transcription factor PAX5.

Using a methylated CpG island amplification technique, applicants identified these two genes, PAX5 α and β, that exhibit promoter hypermethylation in common cancers such as breast, colon and lung. These genes, located on chromosome 9p13, are transcribed from two distinct promoters and result in the formation of two alternative 5' exons spliced to the common exons 2-10. The resulting splice variants encode two distinct transcription factors important in cell differentiation and embryonic development. Recent evidence by others now demonstrates that expression of these genes is deregulated in several tumor types; however, no one has implemented promoter hypermethylation as a means for silencing these genes.

The present invention exploits the determination that the PAX5 α, and β genes are both frequent targets for aberrant methylation in tumor cell lines, as well as primary tumors from breast, lung, and colon. The invention includes the following PAX5 methylation specific polymerase chain reaction (MSP) primer sequences to assay for promoter methylation:

| **Stage 1 MSP Primers** | | |
|---|---|---|
| **Gene** | **Primers (5' - 3')** | **Product Size** |
| PAX5 alpha | gggtttgtatatggagatgttatagg | |
| | caacatcacaaaatatccccaaacac | 389 bp |
| PAX5 beta | agtttgtgggttgtttagttaatgg | |
| | caaaaaatcccaaccaccaaaacc | 328 bp |

| **Stage 2 MSP Primers** | | |
|---|---|---|
| **Gene** | **Primers (5' - 3')** | **Product Size** |
| PAX5 alpha | ataaaagtttggggcggcgc | |
| | gcgcccccaacgcgccg | 166 bp |
| PAX5 beta | gagttgagtttcgggcggc | |
| | gccgccgccgccgtcg | 124 bp |

Using the MSP assay, applicants examined the methylation status of the Pax5 α and β genes in breast, lung, and colon cancers. Pax5 α is methylated in approximately 70% of breast and lung tumors and 13% of colon tumors. The Pax5 β gene is methylated in approximately 60% of breast and lung tumors and 20% of colon tumors.

Accordingly, the present methods find beneficial use, in association with the techniques disclosed in U.S. Patent No. 6,017,704 and PCT Application No. PCT/US01/26452, for the early detection of cancer, monitoring tumor progression, monitoring the response to radio- and chemotherapy, predicting disease outcome, and monitoring response to prevention therapy. The methods improve upon known cancer screening technology because they provide additional markers for early detection, monitoring of therapy, and prediction of disease outcome.

An initial step was the identification of PAX5 α and β hypermethylation. The MCA/RDA technique developed by Toyota *et al.,* was used to identify genes methylated in breast cancer **(Toyota *et al.,*1999).** A subtractive library was constructed, and 100 clones were analyzed by DNA sequencing. Comparison of these clones revealed 50 unique sequences, and a Blast search of each clone confirmed that 48 of 50 clones were homologous to Genbank sequences located in the high-throughput genomic sequence database. One of these clones was identical to the 5' flanking region and exon 1 β of the transcription factor PAX5 (Genbank accession # AF074913). Analysis of the genomic structure of this gene revealed the presence of two distinct promoters resulting in two alternative 5' exons (1α and 1 β) that are spliced to common exons 2-10 resulting in the translation of two unique proteins due to a frameshift **(see Busslinger *et al.,*1996).** Inspection of the α and β promoters revealed that each region is representative of a CpG island **(see Gardiner-Garden and Fromme, 1987; Antequera and Bird, 1993)**, with a GC content of 0.68 and 0.69; a CpG/GpC ratio of 0.70 and 0.73; and a total of 53 and 162 CpG sites in a 520 bp and 1,780 kb region, respectively.

A MSP analysis of the PAX5 α and β genes was then undertaken. Combined bisulfite restriction analysis (COBRA) analysis (8) was initially conducted on DNA from ten breast cell lines, five lung cancer cell lines, and five normal lymphocytes to screen for aberrant methylation of the PAX5 α and β genes. Following bisulfite modification, PCR products were produced using stage-1 primers that do not discriminate between methylated and unmethylated alleles. Methylated alleles were detected in the PAX5 α (389 bp) and β (328 bp) products following digestion with the restriction enzyme BSTU1, which specifically cleaves at CGCG sites that are retained following bisulfite modification due to the presence of methylated CpGs. Complete digestion of the PCR products indicative for methylation of the PAX5 α gene was observed in seven breast and five lung cancer cell lines. The PAX5 α PCR product was partially digested (10-80%) for two breast and lung cancer cell lines. Similarly, the PAX5 β gene was completely digested in five breast and three lung cancer cell lines. The PAX5 β PCR product was also partially digested (10-80%) for two breast and one lung cancer cell lines. PCR products from normal lymphocytes remained undigested, suggesting a lack of methylation (data not shown).

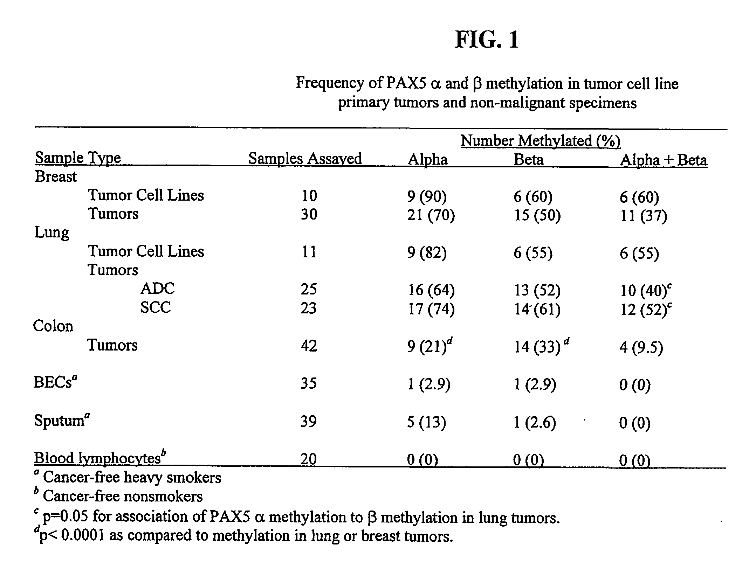

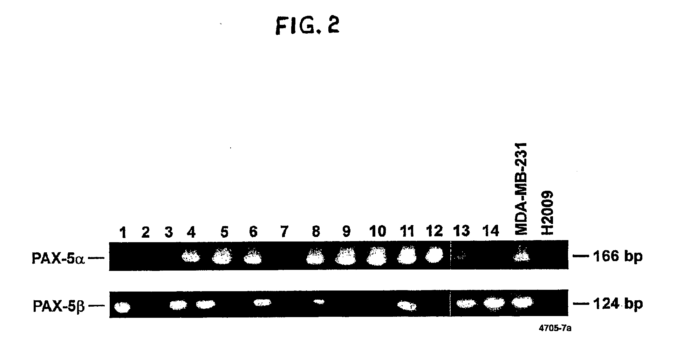

The frequency of PAX5 α and P methylation was then characterized in a panel of primary tumors (breast, lung, and colon) using applicants' two-stage MSP approach (see Patent Cooperation Treaty Application No. PCT/US01/26452, filed August 24, 2001). The results are summarized in Fig. 1, and a representative MSP analysis is shown in Fig. 2 for both the PAX5 α and PAX5 P genes. Fig. 2 depicts MSP analysis of the PAX5 α and P genes in a panel of primary breast tumors. Bisulfite-modified DNA was amplified with stage-2 primers specific for methylated alleles. The MDA-MB-231 and H2009 represent positive and negative cell lines for methylation of both the PAX5 α and β genes, respectively. In both breast and lung tumors, the frequency of PAX5 α methylation approximated 70% and tended to be greater than that observed for the PAX5 β gene (54%). The frequency of PAX5 α and β methylation in colorectal tumors was significantly less than that in either lung or breast tumors (p < 0.0001), but methylation of the β gene was strongly associated (p = 0.05) with methylation of the α gene in lung tumors and cell lines (Fig. 1).

MSP analysis for methylation in cell lines corroborated that seen by the COBRA assay. Overall, 90% and 60% of breast cancer-derived cell lines were methylated for the PAX5 α and β genes, respectively. Eleven lung tumor-derived cell lines were analyzed by MSP, and 82% and 55% were methylated for the PAX5 α and β genes, respectively. Methylated alleles were also detected at a lower prevalence (3-13%) in cultured nonmalignant bronchial epithelial cells (BEC) and sputum from cancer-free, high-risk subjects. Neither PAX gene was methylated by MSP in blood lymphocytes from nonsmokers.

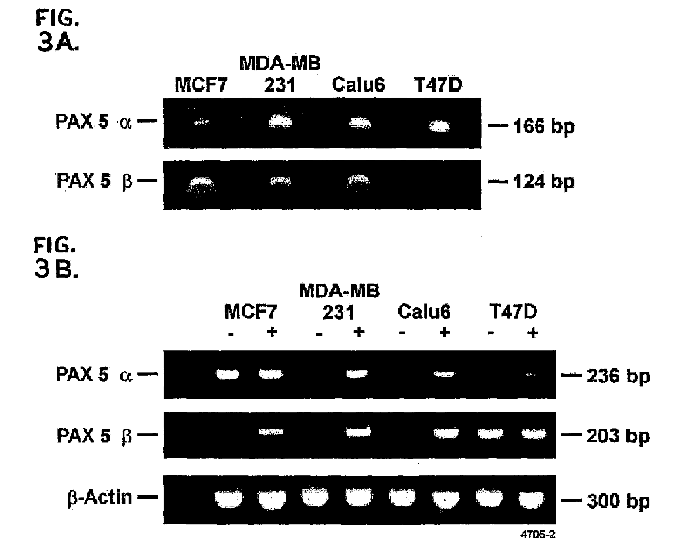

Reverse transcription-PCR (RT-PCR) was conducted on cDNA from three breast cancer cell lines (MCF7, MDA-MB 231, and T47D) and one lung cancer cell line (Calu6) to determine the relationship between methylation of the PAX5 α and β genes and transcription. COBRA analysis demonstrated complete digestion for the PAX5 α gene in the MDA-MB-231 and T47D cell lines and partial digestion for MCF7 (20%) and Calu6 (80%). For the PAX5 β gene, complete digestion was seen in the MCF7, MDA-MB 231, and Calu6 cell lines, but no digestion for T47D. Reference is made to Fig. 3A, showing an MSP analysis of breast and lung cancer cell lines. Bisulfite-modified DNA was amplified with stage-2 primers specific for methylated alleles. Fig. 3B shows an RT-PCR analysis for expression of the PAX5 α and β genes in breast and lung cancer cell lines, which were grown in the presence (+) and absence (-) of 1 µM DAC for 72 h. Expression of the PAX5 α and β genes was restored in non-expressing cell lines following DAC treatment. Expression of the β-actin gene was determined as a control for RNA integrity. As indicated in Fig. 3A, these methylation changes were then corroborated by MSP. PAX5 α, transcript was abundant in MCF7 and to a lesser extent present in Calu6, while no transcript was present in the MDA-MB 231 and T47D cell lines, as seen in Fig. 3B. Treatment of these cell lines with 1 µM DAC, an inhibitor of DNA methyltransferase, increased expression in Calu6 and restored expression in MDA-MB231 and T47D. PAX5 α transcript was only detected in T47D; however, treatment with DAC restored expression in the other three cell lines. Treatment with DAC did not affect the expression of the housekeeping gene β-actin.

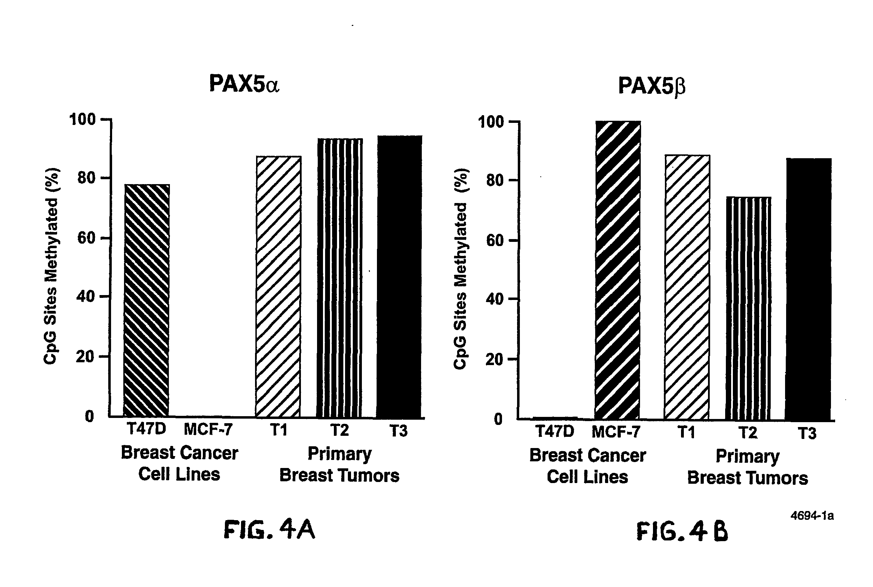

Methylation density within the PAX5 α and β promoter regions amplified by the MSP primers was then determined for the MCF-7 and T47D cell lines and three primary breast tumors. The PCR primers used for the COBRA analysis were also used to amplify the modified DNA from the cell lines, while methylation-specific primers were used to detect methylated sequences in the primary tumors where contaminating stromal and inflammatory cells are present. Sixteen and 13 CpG sites spanning the 116 and 86 bp regions between the MSP primers for the PAX5 α and β sequence, respectively, were evaluated for comparing methylation density between cell lines and tumors. Reference is made to Figs. 3A, 3B, and 4A and 4B, illustrating how the sequencing results corroborated the expression studies, with 78% of sites methylated within the PAX5 α promoter in the T47D cell line, and 100% of sites methylated within the PAX5 β promoter in the MCF-7 cell line. Figs. 4A and 4B graphically show the methylation density of the PAX5 α and β genes. Genomic DNA from breast cancer cell lines T47D and MCF-7, and three primary breast tumors were treated with sodium bisulfite. PAX5 α and β PCR products containing 16 and 13 CpG sites, respectively, were generated. The PCR products were cloned, and five individual clones/sample were sequenced. Summary data are presented as the percent of CpG sites methylated. The CpG methylation density in all three primary tumors was very similar to the patterns observed in the methylated cell lines for each gene, consistent with these regions being important sites for methylation and silencing of the PAX5 α and β genes (See Fig. Figs. 4A and 4B).

The PAX gene family consists of nine members, each of which share a common motif called the paired box that displays DNA-binding properties **(Stuart *et al.,*1995).** PAX proteins function as nuclear transcription factors important for cellular differentiation, migration, and proliferation **(Schafer, 1998)**. That these genes are strong transcriptional regulators makes them likely targets for disruption in oncogenesis. Consistent with this premise, inappropriate expression of the PAX5 gene has been implicated in the pathogenesis of small lymphocytic lymphoma cancer and advanced stage glioblastoma **(Schafer, 1998)**. According to the present disclosure, the PAX5 α and β genes are common targets for inactivation by aberrant promoter hypermethylation in lung, breast, and (to a lesser extent) colorectal tumors. Both genes exhibit homogeneous and dense methylation patterns that correlated with loss of transcription: Additionally, methylation of the PAX5 β gene is associated with methylation of the α gene in lung tumors, so there is a selective advantage to target both genes for inactivation in this cancer type.

Previous studies using MCA and RDA identified the PAX6 gene that is involved in eye, nose, pancreas, and brain development as a common target for aberrant methylation in colonic mucosa during aging **(Toyota *et al.,* 1999a; Toyota *et al*., 1999b).** In these studies, the CpG island identified was localized within an enhancer present in the 5' region of the PAX6 gene. A CpG island within the exon 5 coding region of the PAX6 gene was also hypermethylated in bladder and colon tumors **(Salem *et al.,* 2000)**. While methylation of the promoter region and exon 5 was common in cell lines, methylation in primary tumors was largely confmed to exon 5 and did not affect gene transcription. This is in marked contradistinction from the present methods using the PAX5 α and β genes. According to the present methods, dense methylation is seen in both promoter regions in derived cell lines and primary lung and breast cancers that correlated directly with loss of transcription. The difference seen in primary tumors for silencing the PAX5 and PAX6 genes may be linked to their functions in the respective tissues. No expression of the PAX6 gene was seen in normal colonic mucosa, thereby negating any selective advantage for methylation of the promoter region; again in contradistinction, according to the present invention an abundant expression of both PAX5 transcripts in normal lung tissue is observed.

The fact that both PAX5 genes are expressed in normal lung suggests that these proteins are likely also modulating gene activity in pulmonary cells. The short arm of chromosome 9 is a frequent site for loss of heterozygosity in lung cancer, with deletions extending from 9p13-9p21 in approximately 50% of non-small cell lung cancers **(Testa *et al.,* 1994)***.* Both the PAX5 (9p13) and p16 (9p21) genes are localized to this region and now share the commonality of being inactivated frequently in lung cancer by aberrant promoter methylation **(Esteller *et al.,* 2001; Belinsky *et al.,* 1998)**. There is also precedent for inactivating genes that code for transcription factor binding proteins by promoter hypermethylation. Hypermethylation in cancer (HIC-1) is a zinc-fmger transcription factor gene that is commonly expressed in normal tissues, but inactivated by promoter hypermethylation in lung, breast, colon, and hematopoietic tumors **(Wale *et al.,* 1995; Issa *et al.,*1997)**. Similar to the PAX5 gene, HIC1 is also important in development with knockout mice dying perinatally and exhibiting gross developmental defects involving the brain, cleft palate, and limbs **(Carter *et al.,* 2000)**.

Changes in gene-specific methylation may serve as intermediate biomarkers for cancer detection, risk assessment, and monitoring disease in sputum and blood **(Palmisano *et al.,* 2000; Esteller *et al.,* 1999)**. For example, respecting lung cancer, the aberrant methylation of the p16 and/or O⁶-methylguanine-DNA methyltransferase promoters was detected in DNA from sputum of patients with squamous cell carcinoma (SCC) up to three years before clinical diagnosis **(Palmisano *et al.,* 2000)**.

According to the present methods, the PAX5 α and β genes constitute new intermediate markers for evaluation. Although the timing for inactivation of these genes is not precisely defined, the low frequency of methylation seen in bronchial epithelium and sputum from cancer-free, high-risk subjects indicates that these genes may be methylated during progression rather than initiation. This contrasts with applicant's finding with p16, which is inactivated at the earliest cytologic stages of SCC and adenocarcinoma (ADC), a fact that may account for its more common detection (35%) in bronchial epithelium and sputum from current and former smokers. See Patent Cooperation Treaty Application No. PCT/US01/26452, filed August 24, 2001, assigned to the assignee of the present invention. The utility of plasma or serum for detecting circulating aberrantly methylated DNA in patients with colorectal, head and neck, and breast cancers is known **(Zou *et al.,* 2002, Silva *et al.,* 2002)**. Thus, the inclusion of the PAX5 genes into molecular marker panels for lung, breast, and colon cancer improves the sensitivity and specificity for developing risk models for detecting these cancers through analysis of sputum and blood in high-risk subjects.

Thus the PAX5 α and β genes that are inactivated by promoter hypermethylation demonstrate utility as biomarkers for predicting cancer risk. A panel of genes, inactivated by promoter hypermethylation, has been identified as biomarkers for predicting lung cancer risk and for early detection. Identification occurred in a nested, case-control study conducted on subjects recruited through the University of Colorado. The establishment of a cohort of current and former smokers targeting persons with chronic obstructive pulmonary disease (COPD) and smoking histories in excess of 30 pack-years was initiated. Home sputum was collected longitudinally (optimally once a year) on all enrolled subjects. Enrollment in this cohort has now reached 3,000 persons, and approximately 120 incident cases of lung cancer have been observed.

In an initial study, 33 incident cases of lung cancer were selected and matched 1:1 by age, gender, and date of first sputum sample to 33 persons who were still clinically cancer free (controls). Sputum samples collected on controls after the matched case was diagnosed with cancer were censored. Methylation of the PAX5 alpha and beta represented two of the genes assessed in sputum specimens in the laboratory with blinded replicates for quality control and coded identifiers for all subjects such that our laboratory has remained blinded as to case-control status. The number of sputum samples collected per person ranged from one to five specimens. PAX5 alpha and beta methylation in sputum was associated with odds ratio of 2.0 and 2.4, respectively. Thus, these findings suggest that methylation of the PAX5 genes is an important component of a panel of markers for predicting lung cancer risk.

Through a gene discovery program, several other candidates possessing CpG rich promoter regions have emerged as gene targets in cancer. Three CpG islands have been identified, two of which predict for genes, while the other remains to be characterized. The characteristics of each island are shown below.

| CpG Island | %GC Content | Chromosome Location |
|---|---|---|
| 1. Novel helix loop helix binding protein homologous to the murine gene BHLHB4 | 72% | 20q13 |
| 2. Novel gene | 69% | 20q12-13.2 |
| 3. Beta 3 | 72% | 8q |

The first two CpG islands are clustered within chromosomal region 20q13. The novel helix loop helix gene is homologous to BHLHB4, a gene isolated from a mouse pancreatic β-cell line. The murine gene is proposed to modulate the expression of genes required for the differentiation and/or maintenance of pancreatic and neuronal cell types. The Beta 3 gene is a single exon gene that also codes for a basic helix loop helix protein. Applicants' studies to date indicate that all three CpG islands are inactivated by promoter hypermethylation in approximately 80% of breast cancer cell lines evaluated (n = 8-10). In contrast, CpG island 1 is commonly inactivated in lung cancer cell lines (8 of 11) while the prevalence for inactivation of the other two CpG islands in cell lines is <10%.

Applicants have extended studies of CpG island 1 to primary squamous cell carcinoma of the lung and find inactivation by methylation in 60% of tumors evaluated. Thus, these 3 novel genes likely may be involved in the development of breast and/or lung cancer and could be valuable as biomarkers for early detection, prognosis, and monitoring the efficacy of preventive interventions. In addition, these genes may also prove in future studies to be inactivated in other solid and liquid tumors (e.g., leukemia), making them equally valuable as biomarkers for other cancers. The following are the primer sequences and annealing temperatures for the Novel Helix Loop Helix Binding Protein, Novel Gene 2, and Beta 3 Genes.

| **Stage 1 PCR** | | | |
|---|---|---|---|
| **Gene** | **Primers (5' - 3')** | **Annealing Tm** | |
| **Product Size** | | | |
| HLHBP | gagggagaggaggtgggagag crtaaccrtaacttaataccaaatac | 58 | 267 bp |
| Novel Gene 2 | gtttagttyggaggaaggattttta taataataatccaaatacrccaaacc | 60 | 331 bp |
| Beta 3 | aaagaaagaaggggagagggtttt acaacaacaaccctaccccctc | 60 | 393 bp |
| | Y=C+T,R=A+G | | |

| **Stage 2 PCR** | | | |
|---|---|---|---|
| **Gene** | **Primers (5' - 3')** | **Annealing Tm** | |
| **Product Size** | | | |
| HLHP | gaggaggtagcgggcgtc tcgaccataaccgcgccg | 66 | 186 bp |
| Novel Gene 2 | ggtcggaataatagcgcgc gaacgtccataacgaacgcg | 68 | 181 bp |
| Beta 3 | tagtattaggatcgacgcgc gtcctcgccgacgaccg | 68 | 179 bp |

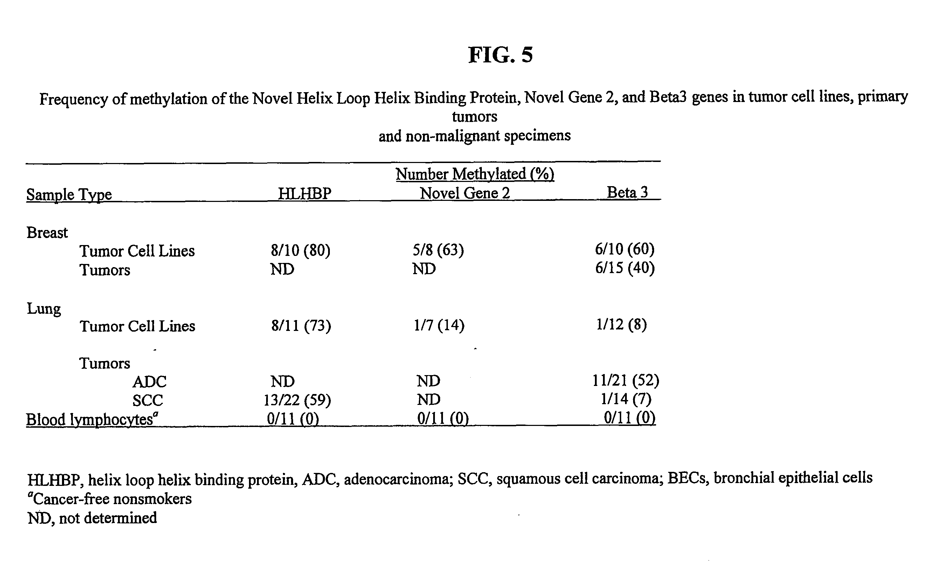

Fig. 5 tabulates the frequency of methylation of the novel Helix Loop Helix Binding Protein, Novel Gene 2, and Beta3 genes in tumor cell lines, primary tumors and non-malignant specimens.

### Industrial Applicability:

The invention is further illustrated by the following non-limiting example.

**Tissue Samples and Cell Lines**. Lung SCCs were obtained from patients previously enrolled in a Lung Cancer Surveillance Study conducted through St. Mary's Hospital, Grand Junction, Colorado. Lung ADCs were acquired from the Johns Hopkins Lung Spore Repository, Baltimore, Maryland. Breast tumors were collected from women enrolled in a New Mexico Women's Health Study being conducted within the Epidemiology and Cancer Control Program at the University of New Mexico. Colorectal carcinoma samples were collected from patients undergoing surgery at the Hospital de Sant Pau and the Hospital de Bellvitge in Barcelona and Catalonia, Spain, respectively. Sputum, nonmalignant BECs, and blood lymphocytes were obtained from veterans who use the Multispecialty Chest Clinic at the New Mexico Veterans Health Care System for their primary care. Sputum cytology was not diagnostic for cancer in any of these individuals and ranged from normal to marked dysplasia in this population. All subjects gave informed consent according to institutional guidelines. Tumor-derived cell lines were obtained from the American Type Culture Collection and were cultured according to their conditions.

**MCA/RDA**. MCA/RDA was performed as described by Toyota *et al.* **(Toyota *et al.*, 1999a)**. Briefly, 5 µg of DNA from the cell line MCF7 was used as the tester, and a mixture of DNA from normal breast tissue of five women (1 µg each) was used as the driver. MCA amplicons were produced using RMCA adaptors, and two rounds of competitive hybridizations were performed. The resulting RDA products were digested with the restriction endonuclease *XmaI* (New England Biolabs) and subsequently cloned into pBluescript KS+.

**DNA Sequencing and Analysis**. Plasmid DNA containing the RDA products was prepared using the QIAprep Spin Miniprep according to the manufacturer's instructions (Qiagen). Virco (Cambridge, UK) analyzed the sequences using an automated DNA sequencer (Applied Biosystems). Sequence homologies were determined using the Blast program of the National Center for Biotechnology Information (http://www.ncbi.nlm.nih.gov/BLAST/).

**MSP and Bisulfite Sequencing**. DNA was isolated by standard phenolchloroform extraction and ethanol precipitation. Genomic DNA was modified by treatment with sodium bisulfite that converts only unmethylated cytosines to uracil. The methylation status of the PAX5 α, and β genes was determined using a nested, two-stage method described previously **(Palmisano *et al.,* 2000)**. Primer sequences used in the stage-1 amplification of each gene are as follows:
α-Forward, (5'-gggt ttgtatatggagatgttatagg-3');
α-Reverse, (5'-caacatcacaaaatatccccaaacac-3');
β-Forward, (5'-agtttgtgggttgtttagttaatgg-3'); and
β-Reverse, (5'-caaaaaatcccaaccaccaaaacc-3').

All PCR amplifications were performed using a Biometra T3 thermocycler and Taq Gold polymerase (Perkin-Elmer). The cycling parameters for stage-1 α were as follows: 94°C for 10 min; then 40 cycles of 94°C for 1 min, 60°C for 1 min, and 72°C for 1 min; and a 5-min final extension at 72°C. Stage-1 P conditions were identical except the annealing temperature was reduced to 54°C, and all cycling times were performed for 30 s. The size of the stage-1 α and β PCR products was 389 and 328 bp, respectively. Primer sequences used in the stage-2 amplification of each gene are as follows:
α-BSM1, (5'-ataaaagtttggggcggcgc-3');
α-BSM2, (5'-gcgcccccaacgcgccg-3');
β-BSM1, (5'-gagttgagtttcgggcggc-3'); and
β-BSM3, (5'-gccgccgccgccgtcg-3').

The cycling parameters for stage-2 α were as follows: 94°C for 10 min; then 40 cycles of 94°C for 15 s, 66°C for 15 s, and 72°C for 15 s; and a 5-min final extension at 72°C. Stage-2 β conditions were identical except the annealing temperature was decreased to 64°C. The size of the stage 2-α and β PCR products was 166 and 124 bp, respectively.

DNA isolated from cell lines MDA-MB-231 and NIH-2009 served as positive and negative controls, respectively, for both genes. All assays were conducted in at least duplicate and were confirmed positive for methylation by restriction digestion with *Bst*UI or by DNA sequencing.

Bisulfite-modified DNA from the T47D and MCF-7 cell lines was amplified using the stage-1 primers for the PAX5 α, and β genes. Stage-1 and methylation-specific stage-2 primers were used to amplify modified DNA from three breast tumors shown to be methylated for the α or β gene. The PCR products were ligated into the PCR II vector using the TA cloning kit (Invitrogen, San Diego, California). Five clones from each sample were sequenced.

**5-Aza-2'-deoxycytidine Treatment and RT-PCR**. Re-expression studies were performed using three breast cancer cell lines (MCF7, MDA-MB-231, and T47D) and one lung cancer cell line (Calu6). Cell lines were treated for 3 days in culture media with 1 µM DAC (Sigma Chemical Co.). Total RNA was prepared using Trizol (Life Technologies, Inc.), and 3 µg aliquots were reverse-transcribed using the Superscript kit (Life Technologies, Inc.). The expression of the PAX5 α and β transcripts were determined by reverse transcription (RT) -PCR using the exon 1α forward primer (5'-cctgtccattccatcaagtcctg-3') and the exon 1β forward primer (5'-cccgatggaaatacactgtaagcac-3') with an exon 2 reverse primer (5'-ttttgctgacacaaccatggctgac-3'). β-actin was also amplified as a control for RNA integrity.

PCR amplification was performed at 94°C for 10 min; then 35 cycles of 94°C for 30 s, 64°C for 30 s, and 72°C for 30 s; and a 5-min final extension at 72°C. PCR products were analyzed on a 3% agarose gel containing ethidium bromide and visualized under UV illumination.

The proportion of tumors positive for methylation of PAX5 α or β was compared among cancer types with the Fisher's exact test. The association between PAX5 α and β methylation for each cancer type was also assessed by the Fisher's exact test.

The preceding example can be repeated with similar success by substituting the generically or specifically described reactants and/or operating conditions for those used in the preceding examples.

### SEQUENCE LISTING

### NUMBER OF SEQ ID NOS: 10

SEQ ID NO 1
   LENGTH: 52
   TYPE: DNA
   ORGANISM: HOMO SAPIENS
   SEQUENCE: 1
   gggtttgtat atggagatgt tataggcaac atcacaaaat atccccaaac ac 52
SEQ ID NO 2
   LENGTH: 49
   TYPE: DNA
   ORGANISM: HOMO SAPIENS
   SEQUENCE: 2
   agtttgtggg ttgtttagtt aatggcaaaa aatcccaacc accaaaacc 49
SEQ ID NO 3
   LENGTH: 37
   TYPE: DNA
   ORGANISM: HOMO SAPIENS
   SEQUENCE: 3
   ataaaagttt ggggcggcgc gcgcccccaa cgcgccg 37
SEQ ID NO 4
   LENGTH: 35
   TYPE: DNA
   ORGANISM: HOMO SAPIENS
   SEQUENCE: 4
   gagttgagtt tcgggcggcg ccgccgccgc cgtcg 35
SEQ ID NO 5
   LENGTH: 47
   TYPE: DNA
   ORGANISM: HOMO SAPIENS
   SEQUENCE: 5
   gagggagagg aggtgggaga gcrtaaccrt aacttaatac caaatac 47
SEQ ID NO 6
   LENGTH: 51
   TYPE: DNA
   ORGANISM: HOMO SAPIENS
   SEQUENCE: 6
   gtttagttyg gaggaaggat ttttataata ataatccaaa tacrccaaac c 51
SEQ ID NO 7
   LENGTH: 46
   TYPE: DNA
   ORGANISM: HOMO SAPIENS
   SEQUENCE: 7
   aaagaaagaa ggggagaggg ttttacaaca acaaccctac cccctc 46
SEQ ID NO 8
   LENGTH: 36
   TYPE: DNA
   ORGANISM: HOMO SAPIENS
   SEQUENCE: 8
   gaggaggtag cgggcgtctc gaccataacc gcgccg 36
SEQ ID NO 9
   LENGTH: 39
   TYPE: DNA
   ORGANISM: HOMO SAPIENS
   SEQUENCE: 9
   ggtcggaata atagcgcgcg aacgtccata acgaacgcg 39
SEQ ID NO 10
   LENGTH: 37
   TYPE: DNA
   ORGANISM: HOMO SAPIENS
   SEQUENCE: 10
   tagtattagg atcgacgcgc gtcctcgccg acgaccg 37

## Claims

1. (currently amended) A method of monitoring for cancer in a biological specimen containing DNA from cells suspected of being cancerous and having PAX5 β gene-specific promoter methylation comprising the steps of:
subjecting DNA to bisulfite modification;
expanding the number of copies of at least a portion of the PAX5 β gene by a polymerase chain reaction to amplify the portion of the PAX5 β gene where the promoter methylation resides, thereby generating an amplification product; and
using an aliquot of the amplification product generated by the first polymerase chain reaction in a second, methylation-specific, polymerase chain reaction at a temperature of annealing that exceeds the melting temperature of the second primer set to amplify a portion of the gene's CpG island where the promoter methylation resides and detect the presence of inactivation of the PAX5 β gene.

2. (currently amended) A method of monitoring for cancer in a biological specimen containing DNA from cells suspected of being cancerous and having PAX5 α gene-specific promoter methylation, comprising the steps of:
subjecting DNA to bisulfite modification;
expanding the number of copies of at least a portion of the PAX5 α gene by using a polymerase chain reaction to amplify a portion of the PAX5 α gene where the promoter methylation resides, thereby generating an amplification product; and
using an aliquot of the amplification product generated by the first polymerase chain reaction in a second, methylation-specific, polymerase chain reaction at a temperature of annealing that exceeds the melting temperature of the second primer set to amplify a portion of the gene's CpG island where the promoter methylation resides and detect the presence of inactivation of the PAX5 α gene.

3. (new) The method of claim 1 wherein the step of expanding at least a portion of the PAX5 β gene comprises amplifying a 328 base pair fragment with a primer set comprising:
Forward 5' agtttgtgggttgtttagttaatgg
Reverse 5' caaaaaatcccaaccaccaaaacc

4. (new) The method of claim 1 wherein the biological sample from which the DNA is obtained is selected from plasma, mucus, fecal stool, and sputum.

5. (new) The method of claim 2 wherein the step of expanding at least a portion of the PAX5 α gene comprises amplifying a 389 base pair fragment with a primer set comprising:
Forward 5' gggtttgtatatggagatgttatagg
Reverse 5' caacatcacaaaatatccccaaacac

6. (new) The method of claim 2 wherein the biological sample from which the DNA is obtained is selected from plasma, mucus, fecal stool, and sputum.

## Patentansprüche

1. Verfahren zur Krebskontrolle in einer biologischen Probe, welche DNS von Zellen beinhalten, die unter Verdacht stehen, kanzerogen zu sein, und die PAX5 βgenspezifische Promotormethylierungen aufweisen, welches die folgenden Schritte umfasst:
Unterwerfen von DNA einer Bisulfit-Modifikation
Vervielfältigen der Anzahl der Kopien zumindest eines Abschnitts des PAX5 β-Gens mittels einer Polymerasekettenreaktion, um den Abschnitt des PAX5 β-Gens zu amplifizieren, in welchem die Promotormethylierung lokalisiert ist, um somit ein Amplifikationsprodukt zu erzeugen; und
Verwenden eines Aliquots des durch die erste Polymerasekettenreaktion erzeugten Amplifikationsprodukts in einer zweiten, methylierungsspezifischen Polymerasekettenreaktion, bei einer Annealing-Temperatur, welche die Schmelztemperatur des zweiten Primersatzes überschreitet, um einen Abschnitt der CpG-Insel des Gens zu amplifizieren, in der die Promotormethylierung lokalisiert ist, und um das Vorhandendsein einer Inaktivierung des PAX5 β-Gens zu detektieren.

2. Verfahren zur Krebskontrolle in einer biologischen Probe, welche DNS von Zellen beinhalten, die unter Verdacht stehen, kanzerogen zu sein, und die PAX5 αgenspezifische Promotormethylierungen aufweisen, welches die folgenden Schritte umfasst:
Unterwerfen von DNA einer Bisulfit-Modifikation
Vervielfältigen der Anzahl der Kopien zumindest eines Abschnitts des PAX5 α-Gens unter Verwendung einer Polymerasekettenreaktion, um den Abschnitt des PAX5 α-Gens zu amplifizieren, in welchem die Promotormethylierung lokalisiert ist, um somit ein Amplifikationsprodukt zu erzeugen; und
Verwenden eines Aliquots des durch die erste Polymerasekettenreaktion erzeugten Amplifikationsprodukts in einer zweiten, methylierungsspezifischen Polymerasekettenreaktion, bei einer Annealing-Temperatur, welche die Schmelztemperatur des zweiten Primersatzes überschreitet, um einen Abschnitt der CpG-Insel des Gens zu amplifizieren, in der die Promotormethylierung lokalisiert ist, und um das Vorhandendsein einer Inaktivierung des PAX5 α -Gens zu detektieren.

3. Das Verfahren des Anpruchs 1, bei dem der Schritt zur Vervielfältigung zumindest eines Abschnitts des PAX5 β-Gens die Amplifizierung eines 328-Basenpaarfragments mit einem Primersatz, bestehend aus
Forward 5' agtttgtgggttgtttagttaatgg
Reverse 5' caaaaaatcccaaccaccaaaacc
umfasst.

4. Das Verfahren des Anspruchs 1, bei dem die biologische Probe, aus der die DNS erhalten wird, aus Plasma, Schleim, Faeces (Stuhl) und Sputum ausgewählt ist.

5. Das Verfahren des Anpruchs 2, bei dem der Schritt zur Vervielfältigung zumindest eines Abschnitts des PAX5 α-Gens die Amplifizierung eines 389-Basenpaarfragments mit einem Primersatz, bestehend aus
Forward 5' gggtttgtatatggagatgttatagg
Reverse 5' caacatcacaaaatatccccaaacac
umfasst.

6. Das Verfahren des Anspruchs 2, bei dem die biologische Probe, aus der die DNS erhalten wird, aus Plasma, Schleim, Faeces (Stuhl) und Sputum ausgewählt ist.

## Revendications

1. Procédé de suivi pour le cancer dans un échantillon biologique contenant de l'ADN de cellules suspectées d'être cancéreuses et ayant une méthylation du promoteur spécifique du gène PAX5 β, comprenant les étapes consistant à :
soumettre l'ADN à une modification au bisulfite ;
augmenter le nombre de copies d'au moins une partie du gène PAX5 β par une réaction en chaîne par polymérase pour amplifier la partie du gène PAX5 β dans laquelle se situe la méthylation du promoteur, en générant ainsi un produit d'amplification ; et
utiliser une fraction aliquote du produit d'amplification généré par la première réaction en chaîne par polymérase dans une seconde réaction en chaîne par polymérase spécifique de la méthylation à une température d'hybridation qui excède la température de fusion du second jeu d'amorces afin d'amplifier une partie de l'îlot CpG du gène dans laquelle se situe la méthylation du promoteur et de détecter la présence d'une inactivation du gène PAX5 β.

2. Procédé de suivi pour le cancer dans un échantillon biologique contenant de l'ADN de cellules suspectées d'être cancéreuses et ayant une méthylation du promoteur spécifique du gène PAX5 α, comprenant les étapes consistant à :
soumettre l'ADN à une modification au bisulfite ;
augmenter le nombre de copies d'au moins une partie du gène PAX5 α en utilisant une réaction en chaîne par polymérase pour amplifier une partie du gène PAX5 α dans laquelle se situe la méthylation du promoteur, en générant ainsi un produit d'amplification ; et
utiliser une fraction aliquote du produit d'amplification généré par la première réaction en chaîne par polymérase dans une seconde réaction en chaîne par polymérase spécifique de la méthylation à une température d'hybridation qui excède la température de fusion du second jeu d'amorces afin d'amplifier une partie de l'îlot CpG du gène dans laquelle se situe la méthylation du promoteur et détecter la présence d'une inactivation du gène PAX5 α.

3. Procédé selon la revendication 1, dans lequel l'étape d'augmentation d'au moins une portion du gène PAX5 β comprend l'amplification d'un fragment de 328 paires de bases avec un jeu d'amorces comprenant :
directe **5' agtttgtgggttgtttagttaatgg**
inverse **5' caaaaaatcccaaccaccaaaacc**

4. Procédé selon la revendication 1, dans lequel l'échantillon biologique à partir duquel est obtenu l'ADN est choisi parmi le plasma, le mucus, les fèces et le crachat.

5. Procédé selon la revendication 2, dans lequel l'étape d'augmentation d'au moins une portion du gène PAX5 α comprend l'amplification d'un fragment de 389 paires de bases avec un jeu d'amorces comprenant :
directe **5' gggtttgtatatggagatgttatagg**
inverse **5' caacatcacaaaatatccccaaacac**

6. Procédé selon la revendication 2, dans lequel l'échantillon biologique à partir duquel est obtenu l'ADN est choisi parmi le plasma, le mucus, les fèces et le crachat.
